# EUROPEAN PATENT APPLICATION

(11) **EP 1 749 811 A1**
(43) Date of publication of application: **07.02.2007**
(21) Application number: 04771434.0
(22) Date of filing: 09.08.2004
(51) Int. Cl.: C07C 45/35, C07C 47/22, C07C 51/25, C07C 57/05, C07C 45/82, C07C 51/44

(54) **PROCESS FOR PRODUCING (METH)ACROLEIN OR (METH)ACRYLIC ACID**

(30) Priority: 25.05.2004 JP 2004154105
(71) Applicant: MITSUBISHI CHEMICAL CORPORATION, Tokyo 108-0014 (JP)
(72) Inventor: YADA, Shuhei, Yokkaichi-shi, Mie 5108530 (JP); OGAWA, Yasushi, Yokkaichi-shi, Mie 5108530 (JP); TAKASAKI, Kenji, Yokkaichi-shi, Mie 5108530 (JP); SUZUKI, Yoshiro, Yokkaichi-shi, Mie 5108530 (JP)
(74) Representative: Woods, Geoffrey Corlett
(86) International application number: PCT/JP2004/011447
(87) International publication number: WO 2005/115953

(57) **Abstract**

There is provided a process for producing (meth)acrolein or (meth)acrylic acid which is capable of avoiding stoppage of operation of a plant for production thereof as a whole due to failure of an oxidation reaction step in the process and ensuring a continuous stable operation of the plant, and is excellent in economical aspects.

The process for producing (meth)acrolein or (meth)acrylic acid according to the present invention sequentially comprises an oxidation reaction step of subjecting a raw gas to gas-phase catalytic oxidation; a reaction gas cooling step of cooling the resultant reaction gas; a low-boiling fraction separation step of separating low-boiling components from the reaction product; a purification step of separating and removing high-boiling components from the reaction product; and a high-boiling fraction decomposition step of decomposing high-boiling components contained in a bottom liquid obtained from the purification step,
said oxidation reaction step comprising a plurality of oxidation reaction steps which are disposed in parallel with each other and operated at the same time.

## Description

### TECHNICAL FIELD

The present invention relates to a process for producing (meth)acrolein or (meth)acrylic acid, more particularly, to a process for producing (meth)acrolein or (meth)acrylic acid which is capable of avoiding stoppage of operation of a plant for production thereof as a whole due to failure of an oxidation reaction step in the process and ensuring a continuous stable operation of the plant, and is excellent in economical aspects.

### BACKGROUND ARTS

The conventional process for producing (meth)acrolein or (meth)acrylic acid includes an oxidation reaction step of subjecting a raw gas such as propylene, propane and isobutylene to gas-phase catalytic oxidation using molecular oxygen; a reaction gas cooling step of cooling a reaction gas containing the thus obtained (meth)acrolein or (meth)acrylic acid; a low-boiling fraction separation step of separating low-boiling components from the reaction product; a purification step of separating and removing high-boiling components from the reaction product from which the low-boiling components have been separated, to recover the (meth)acrolein or (meth)acrylic acid; and a high-boiling fraction decomposition step of decomposing high-boiling components contained in a bottom liquid obtained from the purification step to recover valuable components and residual (meth)acrolein or (meth)acrylic acid.

In order to ensure a continuous stable operation in the above production process, there have been studied various methods for stabilizing an oxidation catalyst used in the oxidation reaction step. For example, there are known the method of preventing formation of hot spots (abnormal heat generation spots in a catalyst layer) which tend to cause deterioration in yield of the aimed product in a fixed bed multipipe type reactor (for example, refer to Japanese Patent Application Laid-Open (KOKAI) Nos. 7-10802, 8-92147 and 2000-93784 and Japanese Patent Publication (KOKOKU) No. 53-30688), and various methods of filling a catalyst in a reaction tube of a fixed bed reactor (for example, refer to Japanese Patent Application Laid-Open (KOKAI) Nos. 51-127013, 3-294239 and 7-10802).

However, even though the oxidation catalyst used in the oxidation reaction step is improved in itself, the operation condition of the subsequent reaction gas cooling step tends to adversely affect the oxidation reaction step, resulting in adverse influences on the oxidation catalyst. Thus, in order to ensure the continuous stable operation, various improvements in the oxidation reaction step have been conventionally proposed. Nevertheless, at present, these methods have still failed to achieve a continuous stable operation of the process.

Since the (meth)acrolein and (meth) acrylic acid are easily-polymerizable substances, it may be extremely difficult to retain a process liquid in equipments constituting a plant for production thereof upon stopping an operation of the plant unlike ordinary plants for production of other chemical products. For this reason, upon stopping the operation of the plant, in addition to economical loss due to the stoppage, huge time and labor are required to remove the process liquid from the plant and treat the same. Thus, the stoppage of operation of the plant leads to a large economical loss. Therefore, it is extremely important to avoid the stoppage of operation of the plant as a whole due to failure of the oxidation reaction step and ensure a continuous stable operation thereof.

To solve the above problems, it will be considered to adopt such a method of providing, in addition to the main plant, a preliminary plant having substantially the same scale and capacity as those of the main plant, and changing-over the production process from the main plant to the preliminary plant to continue the operation of the process even when the main plant is stopped. However, the provision of the preliminary plant having substantially the same scale which is kept in a non-operated state except for stoppage of the main plant is extremely uneconomical in the consideration of required installation spaces and costs as well as production capacity thereof.

### DISCLOSURE OF THE INVENTION

### PROBLEM TO BE SOLVED BY THE INVENTION

The present invention has been achieved for solving the above conventional problems. An object of the present invention is to provide a process for producing (meth)acrolein or (meth)acrylic acid which is capable of avoiding stoppage of operation of a plant for production thereof as a whole due to failure of an oxidation reaction step in the process and ensuring a continuous stable operation of the plant, and is excellent in economical aspects.

### MEANS FOR SOLVING PROBLEM

As a result of the present inventors' earnest studies for solving the above problems, it has been found that in the process for producing (meth)acrolein or (meth)acrylic acid, when a plurality of oxidation reaction steps are disposed in parallel with each other and operated at the same time, even though the operation of any one series of steps in the process including either one of the oxidation reaction steps is stopped by failure thereof, the operation of the other series of steps can be continued, thereby avoiding stoppage of the plant as a whole.

The present invention has been attained on the basis of the above finding. To accomplish the aim, in a first aspect of the present invention, there is provided a process for producing (meth)acrolein or (meth)acrylic acid which sequentially comprises an oxidation reaction step of subjecting a raw gas to gas-phase catalytic oxidation; a reaction gas cooling step of cooling the resultant reaction gas; a low-boiling fraction separation step of separating low-boiling components from the reaction product; a purification step of separating and removing high-boiling components from the reaction product; and a high-boiling fraction decomposition step of decomposing high-boiling components contained in a bottom liquid obtained from the purification step,

said oxidation reaction step comprising a plurality of oxidation reaction steps which are disposed in parallel with each other and operated at the same time.

### EFFECT OF THE INVENTION

In the process for producing (meth)acrolein or (meth)acrylic acid according to the present invention, since a plurality of oxidation reaction steps are disposed in parallel with each other and operated at the same time, it is possible to avoid stoppage of operation of a plant for production thereof as a whole even though one of the oxidation reaction steps is stopped due to failure thereof, and ensure a continuous stable operation of the plant. Therefore, the process of the present invention is excellent in economical aspects.

### PREFERRED EMBODIMENT FOR CARRYING OUT THE INVENTION

The present invention is described in detail below. The process for producing (meth)acrolein or (meth)acrylic acid according to the present invention comprises an oxidation reaction step of subjecting a raw gas to gas-phase catalytic oxidation; a reaction gas cooling step of cooling the resultant reaction gas; a low-boiling fraction separation step of separating low-boiling components from the reaction product; a purification step of separating and removing high-boiling components from the reaction product; and a high-boiling fraction decomposition step of decomposing high-boiling components contained in a bottom liquid obtained from the purification step.

Acrolein is usually produced from propylene (isobutylene or t-butanol upon production of methacrolein) as a raw material in the presence of a Mo-Bi-based composite oxide catalyst comprising Mo-Bi-Fe-Co-Ni-B-Na-Si-O, etc., and purified by separating low-boiling components such as formaldehyde, acetaldehyde and acetone therefrom. Whereas, acrylic acid may be usually produced by directly using the acrolein produced by the above reaction process (methacrolein is used upon production of methacrylic acid) and subjecting the acrolein to gas-phase catalytic oxidation in the presence of a Mo-V-based composite oxide catalyst comprising Mo-V-Sb-Ni-Cu-Si-O, etc., or produced by subjecting propylene as a raw material to gas-phase catalytic oxidation in the presence of a Mo-Bi-Te-based composite oxide catalyst, a Mo-Bi-Se-based composite oxide catalyst or the like, and purified by separating low-boiling components such as water and acetic acid therefrom. In the following descriptions, the process for producing acrylic acid is explained as a typical example of the process of the present invention. However, the production process of the present invention is also applicable to production of acrolein, methacrolein and methacrylic acid.

### Oxidation reaction step:

The industrial process for producing acrolein and acrylic acid has been conducted, for example, by one-pass method, unreacted propylene recycling method and combustion exhaust gas recycling method. The production process of the present invention may be conducted by any of these methods.

### (1) One-pass method:

The one-pass method is such a method including a front stage reaction into which a mixture of propylene, air and steam is fed to convert the mixed gas into mainly acrolein and acrylic acid, and a rear stage reaction into which the resultant outlet gas from the front stage reaction is fed without separating the above reaction products therefrom. At this time, additional amounts of air and steam required for the rear stage reaction may be generally fed together with the outlet gas from the front stage reaction to the rear stage reaction.

### (2) Unreacted propylene recycling method:

In the unreacted propylene recycling method, an acrylic acid-containing reaction gas obtained in the rear stage reaction is introduced into an acrylic acid-collecting apparatus to collect the acrylic acid in the form of an aqueous solution, and a part of an exhaust gas containing unreacted propylene which is obtained in the collecting apparatus is fed to the front stage reaction to recycle a part of the unreacted propylene.

### (3) Combustion exhaust gas recycling method:

The combustion exhaust gas recycling method is such a method in which an acrylic acid-containing reaction gas obtained in the rear stage reaction is introduced into the acrylic acid-collecting apparatus to collect the acrylic acid in the form of an aqueous solution, and then a whole amount of the exhaust gas from the collecting apparatus is catalytically combustion-oxidized to convert unreacted propylene or the like contained in the exhaust gas into mainly carbon dioxide and water and a part of the thus obtained combustion exhaust gas is added to the front stage reaction.

Examples of the reactor used in the oxidation reaction step may include fixed bed multipipe type reactors, fixed bed plate type reactors and fluidized bed type reactors, though it is not limited to these reactors. Among these reactors, the fixed bed multipipe type reactors have been extensively used to produce acrolein or acrylic acid by gas-phase oxidation reaction of propylene or isobutylene using molecular oxygen or a molecular oxygen-containing gas in the presence of a composite oxide catalyst. The fixed bed multipipe type reactors are not particularly restricted as long as these reactors are ordinarily usable in industrial applications.

In the production process of the present invention, a plurality of oxidation reaction steps are disposed in parallel with each other and operated at the same time. With this arrangement, even when one series of steps in the process including either one of the oxidation reaction steps is stopped due to failure thereof, the other operable series of steps can be continuously operated, thereby avoiding stoppage of operation of the plant as a whole. In the case where the process is conducted by operating two series of steps at the same time, the operation capacity of the respective series of steps is usually not less than 20%, preferably 30 to 70% of an operation capacity obtained when the process is conducted by operating a single series of steps solely. The apparatuses used in the respective series of steps in the process are preferably identical in operation capacity to each other. In the case where the process is conducted by operating three or more series of steps at the same time, the operation capacity of the respective series of steps is usually not less than 20%, preferably 30 to 40% of an operation capacity obtained when the process is conducted by operating a single series of steps solely. In this case, the apparatuses used in the respective series of steps in the process are also preferably identical in operation capacity to each other. In addition, in the case where the process is conducted by operating the three series of steps at the same time, the respective series of steps may be preferably combined such that a sum of operation capacities of any two series of steps is equal to that of the remaining one series of steps. If the apparatus used in any one series of steps in the process has an operation capacity of less than 20%, in the case where a continuous operation of the process must be kept only by operation of the single series of steps including the apparatus, the operation capacity of the process tends to become too low to be adapted to a minimum operating efficiency thereof as required.

For example, in the case where two series A and B of steps are operated at the same time, the operation capacity of the apparatus used in each of the two series A and B is preferably about 50%, respectively, assuming that the operation capacity obtained when the process is conducted by operating the single series of steps solely is 100%. In this case, if the operation of the series A is stopped, since the remaining series B of steps having substantially the same capacity can be operated continuously, it is possible to avoid stoppage of the plant as a whole even though the operating efficiency of the process is reduced by half. In general, the possibility that the two series of steps are stopped simultaneously will be extremely low. Further, the combination of plural series of steps using apparatuses that are different in operation capacity from each other is also possible. For example, the process may be conducted by operating two series of steps which comprise the series A using an apparatus with an operation capacity of about 40% and the series B using another apparatus with an operation capacity of about 60%. However, in such a combination of plural series of steps using the apparatuses having different operation capacities from each other, the costs required for the apparatuses tend to be high. Further, when the series B including the apparatus having a larger operation capacity are stopped, the process must be conducted by operating the series A including the apparatus having a lower operation capacity only. Therefore, during repair of the series B, the operating efficiency of the process is governed by the lower operation capacity of the series A.

When the process is conducted by disposing three series of oxidation reaction steps at the same time, assuming that the operating efficiency obtained when the process is conducted by operating the single series of steps solely is 100%, there are preferably used the method of operating the series A, B and C each including an apparatus having an operation capacity of about 33 to 34%, or the method using the combination of apparatuses in which two apparatuses for the series A and B each have an operation capacity of about 25%, and one apparatus for the series C has an operation capacity of about 50%. When these methods are adopted, even though one optional series of steps are stopped, the other series of steps can be continuously operated, so that it is possible to ensure a continuous operation of the process with an operating efficiency of not less than about 50%.

The amount of raw materials (including recycled materials) fed to the oxidation reaction steps may be controlled depending upon capacities of apparatuses used in the respective series of steps in the process. When any series of steps are stopped, the raw materials are fed to only the remaining series of steps to be continuously operated by means of a change-over device. In such a case, the amount of the raw materials fed to the remaining series of steps to be continuously operated may be controlled according to the operation capacity thereof.

### Reaction gas cooling step:

The reaction gas obtained in the oxidation reaction step which usually has a temperature of 200 to 300°C is fed to the reaction gas cooling column, if required, after recovering heat therefrom. In the reaction gas cooling column, the reaction gas is cooled and liquefied. A noncondensed gas is discharged from a top of the column, and then a part thereof is recycled to the reaction system whereas a remainder thereof is fed to a facility for conversion into harmless substances and then discharged into atmosphere. Examples of a cooling medium used in the reaction gas cooling column may include water, organic solvents and mixtures thereof. The reaction gas cooling column may be usually provided therein with trays or packing materials. The tray or packing materials used in the reaction gas cooling column are not particularly restricted, and any ordinary trays and packing materials may be suitably used therein. These trays and packing materials may be used in the combination of any two or more kinds thereof.

Examples of the trays may include trays having a downcomer such as a bubble-cap tray, a perforate plate tray, a bubble tray, a super-flux tray and a max-flux tray, and trays having no downcomer such as a dual flow tray. Examples of the packing material may include regular packing materials and irregular packing materials. Specific examples of the regular packing materials may include "SULZER PACKING" produced by Sulzer Brothers Limited, "SUMITOMO SULZER PACKING" produced by Sumitomo Heavy Industries Ltd., "MELAPACK" produced by Sumitomo Heavy Industries Ltd., "JEMPACK" produced by Grich Inc., "MONTZPACK" produced by Montz Inc., "GOODROLL PACKING" produced by Tokyo Special Wire Netting Co. Ltd., "HONEYCOMB PACK" produced NGK INSULATORS, LTD., "IMPULSE PACKING" produced NAGAOKA Corporation, and "M. C. PACK" produced MITSUBISHI CHEMICAL ENGINEERING CORPORATION. Specific examples of the irregular packing materials may include "INTERLOCKS SADDLE" produced by Norton Inc., "TERALET" produced by Nittetu Chemical Engineering Ltd., "Pole Ring" produced by BASF AG, "Cascade Mini-Ring" produced by Mass-Transfer Inc., and "FLEXI-RING" produced by JGC CORPORATION.

In the case where the reaction gas cooling step and subsequent steps are conducted in a single series, the minimum operation capacities of the respective apparatuses used therein may be designed so as to cope with such a case where the operations of the oxidation reaction steps are partially stopped. For example, in the case where the operating efficiency of the process is reduced to 50% by the partial stoppage of operations of the oxidation reaction steps, apparatuses capable of operating with an operation capacity of 50% may be respectively used in the reaction gas cooling step and subsequent steps. However, in some cases, the operating capacities of the apparatuses used in the reaction gas cooling step and subsequent steps may fail to correspond to the minimum operating efficiency of the oxidation reaction step. In such cases, any suitable measures may be taken to prevent occurrence of any failure in the series of steps including the oxidation reaction step operated at the higher operating efficiency, or the acrylic acid product obtained in the purification step may be recycled to the low-boiling fraction separation step, the purification step and/or the high-boiling fraction decomposition step in order to correspond to the minimum operating efficiency of the oxidation reaction step.

The above latter method of recycling the acrylic acid product to the respective steps is explained in detail. When the operating efficiency of the oxidation reaction steps is reduced to 40% by stoppage of the oxidation reaction step in one series of the process and when the minimum operating efficiency of at least one of the subsequent steps including the low-boiling fraction separation step, purification step and high-boiling fraction decomposition step is 50%, the amount of the reaction product supplied to the step having a minimum operating efficiency of 50% is short by 10% in terms of operating efficiency. For this reason, the acrylic acid product obtained in the purification step is recycled to the respective steps where the feed amount is short, thereby controlling the feed amount so as to conform to the minimum operating efficiency of the respective steps.

The reaction gas cooling step has a close relationship with the oxidation reaction step. Therefore, the fluctuation of operation of the reaction gas cooling step tends to adversely affect the operation of the oxidation reaction step, thereby causing failure in the catalyst used therein. In particular, when the oxidation reaction step in any series is stopped, the fluctuation of operation of the reaction gas cooling step belonging to the failed oxidation reaction step tends to give adverse influences on the other series of steps including the oxidation reaction steps that are still continuously operated. Further, the low-boiling fraction separation step conducted subsequent to the reaction gas cooling step tends to be fluctuated and suffer from troubles such as polymerization of monomers owing to failures caused in the oxidation reaction step and reaction gas cooling step. For this reason, it is preferred that a plurality of reaction gas cooling steps are disposed in parallel with each other and operated at the same time similarly to the oxidation reaction steps. With this arrangement, even when a certain series of steps in the process is stopped due to failure of the reaction gas cooling step therein, the other operable series of steps can be continuously operated, thereby avoiding stoppage of operation of the plant as a whole.

In the case where the reaction gas cooling steps are respectively provided in plural series of steps in the process, the operation capacities of the respective series of steps as well as the combination method thereof are the same as explained in the oxidation reaction step. Meanwhile, as the method of connecting the plural oxidation reaction steps with the plural reaction gas cooling steps in the respective series, there may be used the method of directly connecting the respective oxidation reaction steps with the corresponding reaction gas cooling steps, or the method of once collecting the plural oxidation reaction steps together prior to introduction into the reaction gas cooling steps, and then dividing the collected product into the plural reaction gas cooling steps in the respective series. More specifically, in the case where the oxidation reaction steps provided in the series A1, A2 ··· An are connected with the reaction gas cooling steps provided in the series B1, B2 ··· Bn, there may be used the method of directly connecting these steps with each other such that the connections A1-B1, A2-B2 ··· An-Bn in the respective series are established, or the method of once collecting the reaction gases from the oxidation reaction steps in the respective series A1, A2 ··· An together, and then dividing the collected reaction gas into the reaction gas cooling steps in the respective series B1, B2 ... Bn.

Among these methods, preferred is the former method (method of directly connecting the corresponding steps with each other in each series). This is because the process constituted from the independent plural series of steps in which the respective oxidation reaction steps are directly connected with the corresponding reaction gas cooling steps can be easily controlled in operation and amounts of raw materials to be fed thereto including recycled materials. As described above, the oxidation reaction step and the reaction gas cooling step are mutually influenced. However, there is rarely caused such a case where the oxidation reaction step and the reaction gas cooling step provided in separate series (for example, A1 and B2) suffer from failure at the same time. Therefore, in the former method, the possibility that the two series of steps in the process are stopped at the same time is extremely low. On the other hand, in the latter method of once collecting the plural oxidation reaction steps and then dividing the collected product into the plural reaction gas cooling steps, a procedure for controlling the respective series of steps tends to be complicated and difficult and, therefore, is not necessarily practical though the method is applicable.

### Low-boiling fraction separation step:

In the low-boiling fraction separation step, low-boiling components such as mainly water and acetic acid are removed from the liquefied reaction product produced in the reaction gas cooling step. Meanwhile, in the production of (meth)acrolein, formaldehyde, acetone and acetaldehyde are separated as the low-boiling components. The removal of the low-boiling components is conducted in a low-boiling fraction separation column. As the low-boiling fraction separation column, there may be used one or more distillation columns generally used in plants for production of chemical products. When two or more columns are used in the low-boiling fraction separation step, water is removed from the liquefied reaction product in a front stage dehydration column, and acetic acid is removed from the liquefied reaction product in a rear stage acetic acid separation column. In addition to water and acetic acid, solvents used in the process such as methyl isobutyl ketone, methyl ethyl ketone, toluene, propyl acetate, ethyl acetate and mixtures of any two or more thereof may be separated from the liquefied reaction product. The low-boiling fraction separation columns may be provided therein with trays and packing materials as explained in the reaction gas cooling column.

The heat exchanger (reboiler) attached to the distillation column for heating a bottom liquid thereof is generally classified into two types, i.e., in-column fitted type and out-of-column fitted type. The type of the reboiler attached to the distillation column is not particularly restricted. Specific examples of the reboiler may include vertical fixed pipe plate type reboilers, horizontal fixed pipe plate type reboilers, U-shaped pipe type reboilers, double pipe type reboilers, spiral type reboilers, pyramidal block type reboilers, plate type reboilers and thin-film evaporator type reboilers.

The materials of various nozzles, column body, reboilers, conduits and collision plates (including top plates) of the distillation column are not particularly restricted, and may be appropriately selected according to corresponding liquid properties in view of easily-polymerizable compounds to be treated, temperature conditions and anti-corrosion property. In the production of (meth)acrylic acid, examples of the materials may include stainless steels such as SUS304, SUS304L, SUS316, SUS316L, SUS317, SUS317L and SUS327, and hastelloys.

Since acrylic acid is an easily-polymerizable compound, the low-boiling components are preferably removed from the reaction solution by adding a polymerization inhibitor thereto. Examples of the polymerization inhibitor may include copper acrylate, copper dithiocarbamates, phenol compounds and phenothiazine compounds. Specific examples of the copper dithiocarbamates may include copper dialkyldithiocarbamates such as copper dimethyldithiocarbamate, copper diethyldithiocarbamate, copper dipropyldithiocarbamate and copper dibutyldithiocarbamate; copper cyclic alkylenedithiocarbamates such as copper ethylenedithiocarbamate, copper tetramethylenedithiocarbamate, copper pentamethylenedithiocarbamate and hexamethylenedithiocarbamate; and copper cyclic oxydialkylenedithiocarbamates such as oxydiethylenedithiocarbamate. Specific examples of the phenol compounds may include hydroquinone, methoquinone, pyrogallol, catechol, resorcin, phenol and cresol. Specific examples of the phenothiazine compounds may include phenothiazine, bis-(α-methylbenzyl)phenothiazine, 3,7-dioctyl phenothiazine and bis-(α-dimethylbenzyl)phenothiazine. These compounds may be used singly or in combination of any two or more thereof.

When the low-boiling fraction separation step is conducted in one series, as explained in the reaction gas cooling step, the minimum operation capacity of the apparatus used in the low-boiling fraction separation step may be designed so as to cope with such a case where the oxidation reaction steps and/or the reaction gas cooling steps are partially stopped. If the minimum operation capacity of the low-boiling fraction separation step does not correspond to that of the oxidation reaction step, as explained in the reaction gas cooling step, the acrylic acid product obtained in the purification step is recycled to the low-boiling fraction separation step to conform the minimum operation capacities of these steps with each other.

In the low-boiling fraction separation step, water and acetic acid are mainly separated from the reaction solution. Thus, since the distillation of acrylic acid is performed in the presence of water, solids tend to be produced by polymerization even though any polymerization inhibitor is added thereto. In particular, in the case where the low-boiling fraction separation step is conducted using a single distillation column from the viewpoint of simplicity, an excessive load tends to be applied to the distillation column. For this reason, it is preferred that not only the plural oxidation reaction steps and the plural reaction gas cooling steps but also the plural low-boiling fraction separation steps are respectively disposed in parallel with each other and operated at the same time. More specifically, the oxidation reaction step, reaction gas cooling step and low-boiling fraction separation step which are most likely to suffer from operational failure and stoppage of operation thereof are provided in plural series of steps. With this arrangement, even though one series of steps in the process is stopped, the other series of steps can be continuously operated, thereby avoiding stoppage of operation of the plant as a whole. In the case where the low-boiling fraction separation steps are provided in plural series of steps, the operation capacities of the respective low-boiling fraction separation steps as well as the combination method thereof are the same as explained in the oxidation reaction step. In addition, the connecting method of these steps are the same as explained in the reaction gas cooling step.

Although the low-boiling fraction separation step may be performed using a single distillation column as described above, in order to disperse the load applied to the distillation column and eliminate troubles due to production of solids by polymerization, the low-boiling fraction separation step is preferably divided into a first low-boiling fraction separation step and a second low-boiling fraction separation step in which low-boiling components separated in the first low-boiling fraction separation step are different from those separated in the second low-boiling fraction separation step. More specifically, in the first low-boiling fraction separation step located on the side of the reaction gas cooling step, water is mainly separated from the reaction solution, whereas in the second low-boiling fraction separation step located on the side of the purification step, acetic acid is mainly separated from the reaction solution. In this case, as described above, not only the plural oxidation reaction steps and the plural reaction gas cooling steps but also the plural first low-boiling fraction separation steps and the plural second low-boiling fraction separation steps are preferably disposed in parallel with each other and operated at the same time. However, only the first low-boiling fraction separation step which tends to suffer from troubles due to solids produced by polymerization may be provided in plural series and disposed in parallel with each other. Whereas, the second low-boiling fraction separation step and subsequent steps which can be relatively stably operated may be provided in a single series. This arrangement is advantageous in reducing initial installation costs. The operation capacities of the respective series including the above low-boiling fraction separation steps as well as the combination method thereof are the same as explained in the oxidation reaction step. In addition, the connecting method of the respective series is the same as explained in the reaction gas cooling step.

### Purification step:

In the purification step, high-boiling components are separated from the reaction product from which the low-boiling components have been removed, thereby obtaining a high-purity acrylic acid. Examples of the high-boiling components contained in the reaction product may include maleic anhydride, benzaldehyde, etc. The purification step may be usually performed using a distillation column. Upon the distillation procedure, there may be usually used a polymerization inhibitor. As the polymerization inhibitor, there may be used the same polymerization inhibitors as used in the low-boiling fraction separation step. The high-purity acrylic acid is distilled from a top of the distillation column, and the high-boiling components remain in a bottom liquid thereof.

In the purification step, as explained in the reaction gas cooling step, the minimum operation capacity of the apparatus used therein may be designed to cope with such a case where the oxidation reaction steps and/or reaction gas cooling steps are partially stopped. In order to conform the minimum operation capacity of the apparatus used in the purification step to that used in the oxidation reaction step, as explained in thee reaction gas cooling step, the acrylic acid product obtained in the purification step may be recycled to the low-boiling fraction separation step and/or the purification step.

### High-boiling fraction decomposition step:

In the high-boiling fraction decomposition step, the high-boiling components contained in the bottom liquid obtained in the purification step are decomposed. From the resultant decomposition product are recovered valuable substances such as polymerization inhibitors and acrylic acid which may be recycled to desired steps and reused therein.

The high-boiling fraction decomposition column may be a vertical or horizontal tank-type column which may be equipped with an agitator, heating facilities and distillation columns, if required. As the heating facilities for temperature control, there may be used any of jacketed-type heaters, inner coil-type heaters and external heat exchangers. The decomposition reaction temperature is usually 110 to 250°C, preferably 120 to 230°C. When the decomposition reaction temperature is in the range of 110 to 150°C, the residence time in the decomposition reaction is as relatively long as usually 10 to 50 hr, and when the decomposition reaction temperature is in the range of 150 to 250°C, the residence time in the decomposition reaction is usually 30 min to 10 hr. The decomposition reaction may be conducted under either a reduced pressure or an ordinary pressure. In addition, the trays or packing materials as explained in the reaction gas cooling column may also be provided within the high-boiling fraction decomposition column.

In the high-boiling fraction decomposition step, as explained in the reaction gas cooling step, the minimum operation capacity of the apparatus used therein may be designed so as to cope with such a case where the oxidation reaction steps and/or the reaction gas cooling steps are partially stopped. In order to conform the minimum operation capacity of the apparatus used in the high-boiling fraction decomposition step to that used in the oxidation reaction step, as explained in the reaction gas cooling step, the acrylic acid product obtained in the purification step may be recycled to the low-boiling fraction separation step, the purification step and/or the high-boiling fraction decomposition step.

### Examples:

The present invention is described in more detail by Examples, but the Examples are only illustrative and not intended to limit the scope of the present invention.

### Example 1:

Acrylic acid was produced using a plant for production of acrylic acid which included sequentially an oxidation reaction step, a reaction gas cooling step, a low-boiling fraction separation step, a purification step and a high-boiling fraction decomposition step and had a production capacity of 100,000 tons per year, and in which the oxidation reaction step-reaction gas cooling step were provided in three series A, B and C, and the low-boiling fraction separation step and subsequent steps were provided in a single series. The production capacity of the series A including the oxidation reaction step-reaction gas cooling step was 25,000 tons per year (25% based on the whole capacity); the production capacity of the series B was 25,000 tons per year (25% based on the whole capacity); and the production capacity of the series C was 50,000 tons per year (50% based on the whole capacity). After the elapse of 10 months from initiation of operation of the plant, the differential pressure of an oxidation reactor used in the series A was raised, and feeding of raw materials thereto became impossible. Therefore, the operation of the series A was stopped. At this time, the operations of the oxidation reaction step-reaction gas cooling step in the series B and C were continued, and further the operational load of the low-boiling fraction separation step and subsequent steps which were operated in a single series was reduced to 75% so as to conform to the operation capacity of the respective series B and C. The plant was operated under the above conditions until restoration of the series A. After completion of restoration of the series A, the operational load of the low-boiling fraction separation step and subsequent steps was returned to 100%. As a result, it was confirmed that stoppage of operation of the plant as a whole was avoided.

### Comparative Example 1:

Acrylic acid was produced using a plant for production of acrylic acid which included sequentially an oxidation reaction step, a reaction gas cooling step, a low-boiling fraction separation step, a purification step and a high-boiling fraction decomposition step and had a production capacity of 25,000 tons per year, and in which all of the steps were provided in a single series. After the elapse of 10 months from initiation of operation of the plant, the differential pressure of an oxidation reactor was raised and feeding of raw materials thereto became impossible. Therefore, the operation of the oxidation reactor was stopped. At this time, the operations of all of the reaction gas cooling step and subsequent steps were inevitably stopped, and the acrylic acid-containing reaction solution was discharged out of the reaction system. It took 10 days until restoration of the oxidation reactor, and the operation of the plant as well as production of the acrylic acid were stopped as a whole during the period.

### Comparative Example 2:

The same procedure as defined in Comparative Example 1 was conducted except that during stoppage of the plant as a whole, the acrylic acid-containing reaction solution was preserved in the reaction system without being discharged therefrom. As a result, it was confirmed that when the oxidation reactor was restored after 10 days, polymers were observed in the reaction solution preserved in the reaction system.

### Example 2:

Acrylic acid was produced using a plant for production of acrylic acid which included sequentially an oxidation reaction step, a reaction gas cooling step, a low-boiling fraction separation step, a purification step and a high-boiling fraction decomposition step and had a production capacity of 75,000 tons per year, and in which the oxidation reaction step-reaction gas cooling step were provided in two series A and B, and the low-boiling fraction separation step and subsequent steps were provided in a single series. The production capacity of the series A including the oxidation reaction step-reaction gas cooling step was 25,000 tons per year (about 33% based on the whole capacity); and the production capacity of the series B was 50,000 tons per year (about 67% based on the whole capacity). After the elapse of 10 months from initiation of operation of the plant, the differential pressure of an oxidation reactor used in the series A was raised, and feeding of raw materials thereto became impossible. Therefore, the operation of the series A was stopped. At this time, the operation of the oxidation reaction step-reaction gas cooling step in the series B was continued, and further the operational load of the low-boiling fraction separation step and subsequent steps which were operated in a single series was reduced to 67% so as to conform to the operation capacity of the series B. The plant was operated under the above conditions until restoration of the series A. After completion of restoration of the series A, the operational load of the low-boiling fraction separation step and subsequent steps was returned to 100%. As a result, it was confirmed that stoppage of operation of the plant as a whole was avoided.

### Example 3:

Acrylic acid was produced using the same apparatus as used in Example 2. After the elapse of 10 months from initiation of operation of the plant, the differential pressure of an oxidation reactor used in the series B was raised, and feeding of raw materials thereto became impossible. Therefore, the operation of the series B was stopped. At this time, the operation of the oxidation reaction step-reaction gas cooling step in the series A was continued. Since the operation capacity of the oxidation reaction step-reaction gas cooling step in the series A was about 33% and the operation capacity of the low-boiling fraction separation step and subsequent steps was 50 to 100%, a part of the acrylic acid product obtained in the purification step was fed to the low-boiling fraction separation step to control the operational load of the low-boiling fraction separation step and subsequent steps to 50%. Under the above conditions, the operation of the plant was continued. After completion of restoration of the series B, the operational load of the low-boiling fraction separation step and subsequent steps was returned to 100%. As a result, it was confirmed that stoppage of operation of the plant as a whole was avoided.

## Claims

1. A process for producing (meth)acrolein or (meth)acrylic acid which sequentially comprises an oxidation reaction step of subjecting a raw gas to gas-phase catalytic oxidation; a reaction gas cooling step of cooling the resultant reaction gas; a low-boiling fraction separation step of separating low-boiling components from the reaction product; a purification step of separating and removing high-boiling components from the reaction product; and a high-boiling fraction decomposition step of decomposing high-boiling components contained in a bottom liquid obtained from the purification step,
said oxidation reaction step comprising a plurality of oxidation reaction steps which are disposed in parallel with each other and operated at the same time.

2. A process according to claim 1, wherein said reaction gas cooling step comprises a plurality of reaction gas cooling steps which are disposed in parallel with each other and operated at the same time.

3. A process according to claim 2, wherein said low-boiling fraction separation step comprises a plurality of low-boiling fraction separation steps which are disposed in parallel with each other and operated at the same time.

4. A process according to claim 3, wherein said low-boiling fraction separation step comprises a first low-boiling fraction separation step and a second low-boiling fraction separation step in which low-boiling components separated in the first low-boiling fraction separation step are different from those separated in the second low-boiling fraction separation step, and said first low-boiling fraction separation step located on a side of reaction gas cooling step comprises a plurality of low-boiling fraction separation steps which are disposed in parallel with each other and operated at the same time.

5. A process according to any one of claims 1 to 4, wherein when the process is conducted by operating the plural series of steps at the same time, an operation capacity of each of the plural series is not less than 20% of an operation capacity obtained when the process is conducted by operating only a single series of steps.

6. A process according to any one of claims 1 to 5, wherein the (meth)acrolein or (meth)acrylic acid obtained in the purification step is recycled to at least one step selected from the first low-boiling fraction separation step, the second low-boiling fraction separation step and the purification step.
